# EUROPEAN PATENT APPLICATION

(11) **EP 3 225 693 A1**
(43) Date of publication of application: **04.10.2017**
(21) Application number: 16162632.0
(22) Date of filing: 29.03.2016
(51) Int. Cl.: C12P 13/02, C08F 20/56

(54) **METHOD FOR PREPARING AQUEOUS ACRYLAMIDE SOLUTIONS**

(71) Applicant: BASF SE, 67056 Ludwigshafen am Rhein (DE)
(72) Inventor: Braun, Michael Guenter, 69123 Heidelberg (DE); Daeuwel, Juergen, 69124 Heidelberg (DE); Oedman, Peter, 69124 Heidelberg (DE); Ghislieri, Diego, 64646 Heppenheim (DE); Kleiner, Matthias, 67161 Goennheim (DE); Kiefer, Michael, 67459 Boehl-Iggelheim (DE)
(74) Representative: Zumstein, Angela

(57) **Abstract**

The present invention relates to methods for preparing aqueous acrylamide solutions having a low acrylic acid concentration, aqueous acrylamide solutions obtainable by such methods, and acrylamide homopolymers or copolymers obtainable by polymerizing such aqueous acrylamide solutions. In addition, the present invention is also directed to methods for producing aqueous acrylamide solution having a reduced acrylic acid concentration.

SEQUENCE LISTING

<110>  BASF SE

<120>  Method for increasing the viscosity of a polyacrlyamide solution

<130>  78351 / B15091

<160>  4

<170>  PatentIn version 3.5

<210>  1
<211>  612
<212>  DNA
<213>  Rhodococcus rhodochrous

<400>  1
gtgagcgagc acgtcaataa gtacacggag tacgaggcac gtaccaaggc gatcgaaacc      60

ttgctgtacg agcgagggct catcacgccc gccgcggtcg accgagtcgt ttcgtactac     120

gagaacgaga tcggcccgat gggcggtgcc aaggtcgtgg ccaagtcctg ggtggaccct     180

gagtaccgca agtggctcga agaggacgcg acggccgcga tggcgtcatt gggctatgcc     240

ggtgagcagg cacaccaaat ttcggcggtc ttcaacgact cccaaacgca tcacgtggtg     300

gtgtgcactc tgtgttcgtg ctatccgtgg ccggtgcttg gtctcccgcc cgcctggtac     360

aagagcatgg agtaccggtc ccgagtggta gcggaccctc gtggagtgct caagcgcgat     420

ttcggtttcg acatccccga tgaggtggag gtcagggttt gggacagcag ctccgaaatc     480

cgctacatcg tcatcccgga acggccggcc ggcaccgacg gttggtccga ggaggagctg     540

acgaagctgg tgagccggga ctcgatgatc ggtgtcagta atgcgctcac accgcaggaa     600

gtgatcgtat ga                                                         612


<210>  2
<211>  203
<212>  PRT
<213>  Rhodococcus rhodochrous

<400>  2

Val Ser Glu His Val Asn Lys Tyr Thr Glu Tyr Glu Ala Arg Thr Lys
1               5                   10                  15


Ala Ile Glu Thr Leu Leu Tyr Glu Arg Gly Leu Ile Thr Pro Ala Ala
            20                  25                  30


Val Asp Arg Val Val Ser Tyr Tyr Glu Asn Glu Ile Gly Pro Met Gly
        35                  40                  45


Gly Ala Lys Val Val Ala Lys Ser Trp Val Asp Pro Glu Tyr Arg Lys
    50                  55                  60

Trp Leu Glu Glu Asp Ala Thr Ala Ala Met Ala Ser Leu Gly Tyr Ala
65                  70                  75                  80

Gly Glu Gln Ala His Gln Ile Ser Ala Val Phe Asn Asp Ser Gln Thr
                85                  90                  95

His His Val Val Val Cys Thr Leu Cys Ser Cys Tyr Pro Trp Pro Val
            100                 105                 110

Leu Gly Leu Pro Pro Ala Trp Tyr Lys Ser Met Glu Tyr Arg Ser Arg
        115                 120                 125

Val Val Ala Asp Pro Arg Gly Val Leu Lys Arg Asp Phe Gly Phe Asp
    130                 135                 140

Ile Pro Asp Glu Val Glu Val Arg Val Trp Asp Ser Ser Ser Glu Ile
145                 150                 155                 160

Arg Tyr Ile Val Ile Pro Glu Arg Pro Ala Gly Thr Asp Gly Trp Ser
                165                 170                 175

Glu Glu Glu Leu Thr Lys Leu Val Ser Arg Asp Ser Met Ile Gly Val
            180                 185                 190

Ser Asn Ala Leu Thr Pro Gln Glu Val Ile Val
        195                 200

<210>  3
<211>  690
<212>  DNA
<213>  Rhodococcus rhodochrous

<400>  3
atggatggta tccacgacac aggcggcatg accggatacg gaccggtccc ctatcagaag      60

gacgagccct tcttccacta cgagtgggag ggtcggaccc tgtcaattct gacttggatg     120

catctcaagg gcatatcgtg gtgggacaag tcgcggttct tccgggagtc gatggggaac     180

gaaaactacg tcaacgagat tcgcaactcg tactacaccc actggctgag tgcggcagaa     240

cgtatcctcg tcgccgacaa gatcatcacc gaagaagagc gaaagcaccg tgtgcaagag     300

atccttgagg gtcggtacac ggacaggaag ccgtcgcgga agttcgatcc ggcccagatc     360

gagaaggcga tcgaacggct tcacgagccc cactccctag cgcttccagg agcggagccg     420

agtttctctc tcggtgacaa gatcaaagtg aagagtatga acccgctggg acacacacgg     480

tgcccgaaat atgtgcggaa caagatcggg gaaatcgtcg cctaccacgg ctgccagatc     540

tatcccgaga gcagctccgc cggcctcggc gacgatcctc gcccgctcta cacggtcgcg     600

ttttccgccc aggaactgtg gggcgacgac ggaaacggga aagacgtagt gtgcgtcgat 660

ctctgggaac cgtacctgat ctctgcgtga 690

<210> 4
<211> 229
<212> PRT
<213> Rhodococcus rhodochrous

<400> 4

Met Asp Gly Ile His Asp Thr Gly Gly Met Thr Gly Tyr Gly Pro Val
1               5                   10                  15

Pro Tyr Gln Lys Asp Glu Pro Phe Phe His Tyr Glu Trp Glu Gly Arg
            20                  25                  30

Thr Leu Ser Ile Leu Thr Trp Met His Leu Lys Gly Ile Ser Trp Trp
        35                  40                  45

Asp Lys Ser Arg Phe Phe Arg Glu Ser Met Gly Asn Glu Asn Tyr Val
    50                  55                  60

Asn Glu Ile Arg Asn Ser Tyr Tyr Thr His Trp Leu Ser Ala Ala Glu
65                  70                  75                  80

Arg Ile Leu Val Ala Asp Lys Ile Ile Thr Glu Glu Glu Arg Lys His
                85                  90                  95

Arg Val Gln Glu Ile Leu Glu Gly Arg Tyr Thr Asp Arg Lys Pro Ser
            100                 105                 110

Arg Lys Phe Asp Pro Ala Gln Ile Glu Lys Ala Ile Glu Arg Leu His
        115                 120                 125

Glu Pro His Ser Leu Ala Leu Pro Gly Ala Glu Pro Ser Phe Ser Leu
    130                 135                 140

Gly Asp Lys Ile Lys Val Lys Ser Met Asn Pro Leu Gly His Thr Arg
145                 150                 155                 160

Cys Pro Lys Tyr Val Arg Asn Lys Ile Gly Glu Ile Val Ala Tyr His
                165                 170                 175

Gly Cys Gln Ile Tyr Pro Glu Ser Ser Ser Ala Gly Leu Gly Asp Asp
            180                 185                 190

Pro Arg Pro Leu Tyr Thr Val Ala Phe Ser Ala Gln Glu Leu Trp Gly
        195                 200                 205

Asp Asp Gly Asn Gly Lys Asp Val Val Cys Val Asp Leu Trp Glu Pro
    210                 215                 220

Tyr Leu Ile Ser Ala
225

## Description

### Field of the invention

The present invention relates to methods for preparing aqueous acrylamide solutions having a low acrylic acid concentration, aqueous acrylamide solutions obtainable by such methods, and acrylamide homopolymers or copolymers obtainable by polymerizing such aqueous acrylamide solutions. In addition, the present invention is also directed to methods for producing aqueous acrylamide solution having a reduced acrylic acid concentration.

### Background of the invention

Polyacrylamide is widely used as flocculants, as thickener in the paper industry, as additive in tertiary oil recovery, and many other fields. The raw material for polyacrylamide is typically its monomer acrylamide. In principal, there exist two different methods to produce acrylamide in industrial scales: Chemical synthesis and biological synthesis, wherein the biological synthesis methods are more and more on the rise due to milder reaction conditions and inherent process safety. Due to the milder reaction conditions, the absence of copper catalyst and the quantitative conversion of the nitrile, expensive downstream processing steps such as distillation or ion exchange can be avoided in the biological synthesis, thus resulting in cheaper plants with drastically reduced plant footprint.

Both synthesis methods use acrylonitrile as starting substance. While the chemical synthesis method uses copper catalysts (e.g., US4048226, US3597481), the biological synthesis method (also known as bio-based method) employs biocatalysts to hydrate (*i.e.* to convert) acrylonitrile in order to obtain acrylamide. Generally, such biocatalysts are microorganisms which are able to produce (*i.e.* which encode) the enzyme nitrile hydratase (IUBMB nomenclature as of September 30, 2014: EC 4.2.1.84; CAS-No. 2391-37-5; also referred to as, e.g., NHase). Nitrile hydratase producing microorganisms are largely distributed in the environment and comprise, *inter alia,* representatives of the species *Rhodococcus rhodochrous, Rhodococcus pyridinovorans, Rhodococcus erythropolis, Rhodococcus equi, Rhodococcus ruber, Rhodococcus opacus, Aspergillus niger, Acidovorax avenae, Acidovorax facilis, Agrobacterium tumefaciens, Agrobacterium radiobacter, Bacillus subtilis, Bacillus pallidus, Bacillus smithii, Bacillus sp BR449, Bradyrhizobium oligotrophicum, Bradyrhizobium diazoefficiens, Bradyrhizobium japonicum, Burkholderia cenocepacia, Burkholderia gladioli, Klebsiella oxytoca, Klebsiella pneumonia, Klebsiella variicola, Mesorhizobium ciceri, Mesorhizobium opportunistum, Mesorhizobium* sp F28, *Moraxella, Pantoea endophytica, Pantoea agglomerans, Pseudomonas chlororaphis, Pseudomonas putida, Rhizobium, Rhodopseudomonas palustris, Serratia liquefaciens, Serratia marcescens, Amycolatopsis, Arthrobacter, Brevibacterium sp CH1, Brevibacterium sp CH2, Brevibacterium sp R312, Brevibacterium imperiale, Corynebacterium nitrilophilus, Corynebacterium pseudodiphteriticum, Corynebacterium glutamicum, Corynebacterium hoffmanii, Microbacterium imperiale, Microbacterium smegmatis, Micrococcus luteus, Nocardia globerula, Nocardia rhodochrous, Pseudonocardia thermophila, Trichoderma, Myrothecium verrucaria, Aureobasidium pullulans, Candida famata, Candida guilliermondii, Candida tropicalis, Cryptococcus flavus, Cryptococcus sp* UFMG- Y28, *Debaryomyces hanseii, Geotrichum candidum, Geotrichum* sp JR1, *Hanseniaspora, Kluyveromyces thermotolerans, Pichia kluyveri, Rhodotorula glutinis, Comomonas testosteroni, Pyrococcus abyssi, Pyrococcus furiosus, and Pyrococcus horikoshii.* (see, e.g., Prasad, Biotechnology Advances (2010), 28(6): 725-741; FR2835531). The enzyme nitrile hydratase is either iron- or cobalt-dependent (i.e. it possesses either an iron or a cobalt atom coordinated in its activity center) which is particularly characterized by its ability to catalyze bioconversion of acrylonitrile to obtain acrylamide by hydrating acrylonitrile (Kobayashi, Nature Biotechnology (1998),16: 733 - 736).

The product of a biological synthesis method of converting acrylonitrile to acrylamide is a solution of acrylamide in water. However, in general the obtained aqueous acrylamide solution further contains acrylic acid, which is formed as a byproduct during the bioconversion.

Acrylamide is used as a monomer to form polymers of acrylamide. For the polymerization reactions, aqueous acrylamide solutions, which have been prepared by a biological synthesis method, can be used.

However, it has been found that acrylic acid, which is present in the aqueous acrylamide solutions used for the polymerization reactions, leads to reduced performance of the resulting acrylamide polymers. More specifically, the presence of acrylic acid can significantly impair the physical properties of the acrylamide polymer material, which e.g. leads to a reduced solubility and performance in various applications such as water treatment, paper making, oil recovery or mining.

Thus, there is a need for biocatalytic methods of preparing aqueous acrylamide solutions having a low concentration of acrylic acid.

This objective technical problem has been overcome by the present invention as defined in the claims and as described and exemplified herein below.

### Summary of the invention

Therefore a first aspect of the invention relates to a method for producing acrylamide from acrylonitrile in an aqueous solution using a biocatalyst, wherein the ratio of the total amount of NHase fed to the reaction mixture by the addition of the biocatalyst to the total amount of acrylonitrile fed to the reaction mixture at the end of the reaction is equal or less than 200 U/g acrylonitrile.

It has been surprisingly found that reducing the amount of biocatalyst in the reaction leads to the decrease in the amount of acrylic acid which is formed as a byproduct during the bioconversion of acrylonitrile to acrylamide.

In specific embodiments the ratio of the total amount of NHase fed to the reaction mixture by the addition of the biocatalyst to the total amount of acrylonitrile fed to the reaction mixture at the end of the reaction is equal or less than 188 U/g, preferably equal or less than 174 U/g, most preferably equal or less than 161 U/g acrylonitrile.

The acrylic acid concentration of the reaction mixture at the end of the reaction according to the invention may be 1000 ppm or less, preferably less than 900 ppm or less, more preferably 750 ppm or less, even more preferably 650 ppm or less, most preferably 500 ppm or less based on the total amount of acrylamide produced in the reaction mixture.

Typically, the reaction is carried out in fed-batch mode and wherein acrylonitrile is added to the reaction solution intermittently or continuously.

In specific embodiments, the biocatalyst is treated by a drying step before being added to the reaction mixture. In preferred embodiments, the drying step is a spray drying step or freeze drying step, preferably spray drying step.

In other embodiments a saccharide is added to the reaction mixture.

In further embodiments the biocatalyst may be contacted with urea.

In some embodiments, acrylonitrile is added to the reaction mixture independently of the acrylonitrile concentration in the reaction mixture.

Typically, the final acrylamide content in the reaction mixture is 40 w/w % to 60 w/w , preferably 45 w/w % to 60 w/w %, most preferably at least 50 w/w % to 60 w/w % based on the total weight of the reaction mixture.

In some embodiments, the biocatalyst is selected from the group consisting of *Rhodococcus, Aspergillus, Acidovorax, Agrobacterium, Bacillus, Bradyrhizobium, Burkholderia, Escherichia, Geobacillus, Klebsiella, Mesorhizobium, Moraxella, Pantoea, Pseudomonas, Rhizobium, Rhodopseudomonas, Serratia, Amycolatopsis, Arthrobacter, Brevibacterium, Corynebacterium, Microbacterium, Micrococcus, Nocardia, Pseudonocardia, Trichoderma, Myrothecium, Aureobasidium, Candida, Cryptococcus, Debaryomyces, Geotrichum, Hanseniaspora, Kluyveromyces, Pichia, Rhodotorula, Comomonas*, and *Pyrococcus,* preferably wherein the biocatalyst is selected from the group consisting of *Rhodococcus, Pseudomonas, Escherichia* and *Geobacillus.* The biocatalyst may be for example *Rhodococcus rhodochrous* or *Rhodococcus pyridinovorans.*

A further aspect refers to a method for producing an acrylamide homopolymer or copolymer comprising the method for producing acrylamide from acrylonitrile in an aqueous solution as described herein and further comprising the step of polymerizing the acrylamide obtained by the method for producing acrylamide from acrylonitrile in an aqueous solution as described herein.

Acrylic acid is an undesired byproduct in the bioconversion of acrylonitrile to acrylamide as it may have a negative impact on downstream processing of acrylamide, e.g. on the production of acrylamide polymers. Since the present invention allows for preparation of aqueous acrylamide solutions having a reduced concentration of acrylic acid, in case that such aqueous acrylamide solutions are used for homopolymerization or copolymerization reactions, also the obtained acrylamide homopolymers or copolymers have a reduced content of acrylic acid. Such acrylamide homopolymers or copolymers exhibit improved physical properties, such as solubility, and performance.

Another aspect of the invention refers to an acrylamide homopolymer or copolymer obtainable by the method for producing an acrylamide homopolymer or copolymer as described herein.

Another aspect refers to an acrylamide homopolymer or copolymer, in particular to an acrylamide homopolymer or copolymer obtainable by the method for producing an acrylamide homopolymer or copolymer as described herein, having an acrylic acid content of 60,000 ppm or less, preferably of 20,000 ppm or less, more preferably of 10,000 ppm or less, even more preferably of 2,000 ppm or less, and most preferably of 1,500 ppm or less, wherein the indications of ppm each relate to weight parts and are each referred to the total weight of the solid acrylamide homopolymer or copolymer..

Another aspect refers to a method for producing aqueous acrylamide solution having a reduced acrylic acid concentration when compared to a reference solution by converting acrylonitrile to acrylamide using a biocatalyst, wherein the ratio of the total amount of NHase fed to the reaction mixture by the addition of the biocatalyst to the total amount of acrylonitrile fed to the reaction mixture at the end of the reaction is less than 200 U/g acrylonitrile, wherein the reference solution is a acrylamide solution which is prepared by the same method but wherein the ratio of the total amount of NHase fed to the reaction mixture by the addition of the biocatalyst to the total amount of acrylonitrile fed to the reaction mixture at the end of the reaction is more than 200 U/g acrylonitrile.

In preferred embodiments the ratio of the total amount of NHase fed to the reaction mixture by the addition of the biocatalyst to the total amount of acrylonitrile fed to the reaction mixture at the end of the reaction is equal or less than 188 U/g, preferably equal or less than 174 U/g, most preferably equal or less than 161 U/g acrylonitrile.

### Detailed description of the invention

A first aspect of the invention refers to a method for producing acrylamide from acrylonitrile in an aqueous solution using a biocatalyst, wherein the ratio of the total amount of NHase fed to the reaction mixture by the addition of the biocatalyst to the total amount of acrylonitrile fed to the reaction mixture at the end of the reaction is equal or less than 200 U/g acrylonitrile.

The ratio of the total amount of NHase fed to the reaction mixture by the addition of the biocatalyst to the total amount of acrylonitrile fed to the reaction mixture at the end of the reaction may be equal or less than 190 U/g, equal or less than 188 U/g, equal or less than 186 U/g, equal or less than 184 U/g, equal or less than 182 U/g, equal or less than 180 U/g, equal or less than 178 U/g, equal or less than 176 U/g, equal or less than 174 U/g, equal or less than 172 U/g, equal or less than 170 U/g, equal or less than 168 U/g, equal or less than 166 U/g, equal or less than 164 U/g, equal or less than 162 U/g, equal or less than 161 U/g, equal or less than 160 U/g, equal or less than 155 U/g, equal or less than 150 U/g acrylonitrile.

In specific embodiments the ratio of the total amount of NHase fed to the reaction mixture by the addition of the biocatalyst to the total amount of acrylonitrile fed to the reaction mixture at the end of the reaction is equal or less than 188 U/g, preferably equal or less than 174 U/g, most preferably equal or less than 161 U/g.

In addition, in any one of the methods described herein the ratio of the total amount of NHase fed to the reaction mixture by the addition of the biocatalyst to the total amount of acrylonitrile fed to the reaction mixture at the end of the reaction is more than 10 U/g, preferably more than 50 U/g, more preferably 75 U/g or more acrylonitrile, most preferably 100 U/g acrylonitrile.

In addition, in any one of the methods described herein the acrylic acid concentration of the reaction mixture at the end of the reaction according to the invention may be 10 ppm or more, optionally 50 ppm or more, optionally 100 ppm or more.

The term "reaction mixture" as used herein refers to an aqueous mixture comprising a biocatalyst and acrylonitrile and/or an acrylamide. In some embodiments, the reaction mixture according to any one of the methods disclosed herein may be created by combining a biocatalyst that has undergone an activation step, an aqueous solution and acrylonitrile. Typically, the biocatalyst catalyzes the bioconversion of the acrylonitrile to acrylamide in the reaction mixture. Thus, the term "reaction mixture" typically refers to a mixture including water, a biocatalyst, acrylonitrile and/or acrylamide, at any time of a bioconversion process, including at the beginning of the reaction, when in an aqueous solution a biocatalyst is first contacted to acrylonitrile, as well as after the bioconversion has been stopped or ended but when the aqueous solution, the biocatalyst and acrylamide and/or acrylonitrile are still present in the mixture. In particular, the term "reaction mixture" refers to the composition in the reactor.

Typically, the final acrylamide content in the reaction mixture is 40 w/w % to 60 w/w , preferably 45 w/w % to 60 w/w %, most preferably at least 50 w/w % to 60 w/w % based on the total weight of the reaction mixture.

The term "final acrylamide content" refers to the acrylamide content at the end of the reaction.

The term "reaction time" as used herein is defined as time between the beginning and the end of the reaction.

The term "end of the reaction" may refer to the point of time when 99.99 % of the total amount of acrylonitrile fed to the reaction mixture has been converted. In other the words the term "end of the reaction" may refer to the point of time when the total amount of acrylonitrile fed to the reaction mixture has been converted so that the remaining acrylonitrile concentration is equal or less than 100 ppm, wherein ppm refers to weight parts based on the total weight of the reaction mixture. The term "end of the reaction" when used herein can in addition or alternatively be understood as the point of time when the desired acrylamide content in the reaction mixture is achieved, i.e. when an acrylamide content of at least 40 w/w %, preferably at least 45 w/w %, more preferably at least 50 w/w %, such as 40 w/w % to 60 w/w %, 45 w/w % to 60 w/w %, 50 w/w % to 60 w/w % based on the total weight of the reaction mixture is achieved.

The "beginning of the reaction" is defined by the point of time in which the (i) biocatalyst capable of converting acrylonitrile to acrylamide, (ii) acrylonitrile and (iii) water are present in a mixture under conditions that allow the bioconversion of the composition.

The term "amount of acrylonitrile fed to the reaction mixture at the end of the reaction" refers to the total amount of acrylonitrile fed to the reaction mixture during the reaction until the end of the reaction. Thus is in other words the invention refers a method for producing acrylamide from acrylonitrile in an aqueous solution using a biocatalyst, wherein the ratio of the total amount of NHase fed to the reaction mixture by the addition of the biocatalyst to the total amount of acrylonitrile fed to the reaction mixture is equal or less than 200 U/g acrylonitrile. The ratio of the total amount of NHase fed to the reaction mixture by the addition of the biocatalyst to the total amount of acrylonitrile fed to the reaction mixture may be equal or less than 190 U/g, equal or less than 188 U/g, equal or less than 186 U/g, equal or less than 184 U/g, equal or less than 182 U/g, equal or less than 180 U/g, equal or less than 178 U/g, equal or less than 176 U/g, equal or less than 174 U/g, equal or less than 172 U/g, equal or less than 170 U/g, equal or less than 168 U/g, equal or less than 166 U/g, equal or less than 164 U/g, equal or less than 162 U/g, equal or less than 161 U/g, equal or less than 160 U/g, equal or less than 155 U/g, equal or less than 150 U/g acrylonitrile.

Another aspect refers to a method for producing an aqueous acrylamide solution having a reduced acrylic acid concentration when compared to a reference solution by converting acrylonitrile to acrylamide using a biocatalyst, wherein the ratio of the total amount of NHase fed to the reaction mixture by the addition of the biocatalyst to the total amount of acrylonitrile fed to the reaction mixture at the end of the reaction is less than 200 U/g acrylonitrile, wherein the reference solution is an acrylamide solution which is prepared by the same method but wherein the ratio of the total amount of NHase fed to the reaction mixture by the addition of the biocatalyst to the total amount of acrylonitrile fed to the reaction mixture at the end of the reaction is more than 200 U/g acrylonitrile.

In preferred embodiments the ratio of the total amount of NHase fed to the reaction mixture by the addition of the biocatalyst to the total amount of acrylonitrile fed to the reaction mixture at the end of the reaction is equal or less than 188 U/g, preferably equal or less than 174 U/g, most preferably equal or less than 161 U/g acrylonitrile.

In particular, the inventors have found that by carrying out any one of the methods of the present invention as described herein, the acrylic acid concentration may be reduced by at least 5 %, preferably by at least 10 %, more preferably by at least 15 %, even more preferably by at least 20 %, and most preferably by at least 25 % compared to a reference method. In this context, the reduction of the acrylic acid concentration as defined in the methods of the present invention is related to the final concentration of acrylic acid contained in an aqueous acrylamide solution prepared by any one of the methods of the present invention (i.e. for example by the addition of the biocatalyst of less than 200 U/g acrylonitrile) compared to the final concentration of acrylic acid contained in an aqueous acrylamide solution not prepared by the methods of the present invention (i.e. for example by the addition of the biocatalyst of more than 200 U/g acrylonitrile).

The term "bioconversion" as used in the context with any one of the methods of the present invention described herein above and below in general denotes a reaction, wherein acrylonitrile is converted to acrylamide in the presence of water and a biocatalyst. The acrylamide is dissolved in the water, such that by any one of the methods described and provided herein an aqueous acrylamide solution is formed. As used herein, the term "composition" includes all components present in the reactor, such as, for example, the biocatalyst, acrylonitrile, acrylamide and water.

As used with regard to any one of the methods described herein above and below, the term "biocatalyst" comprises in particular microorganisms (e.g., bacteria or protozoic eukaryotes) and enzymes which are capable of converting acrylonitrile to acrylamide. Methods for determining the ability of a given biocatalyst (e.g., microorganism or enzyme) to convert acrylonitrile to acrylamide are well known in the art. As an example, in context with any one of the methods of the present invention, the activity of a given biocatalyst to be capable of converting acrylonitrile to acrylamide in the sense of the present invention, i.e. the activity of a given biocatalyst to act as a nitrile hydratase in the sense of the present invention, may be determined as follows: First reacting 100 µl of a cell suspension, cell lysate, dissolved enzyme powder or any other preparation containing the supposed biocatalyst with 875 µl of an 50 mM potassium phosphate buffer (pH 7,0) and 25 µl of acrylonitrile at 25 °C on an eppendorf tube shaker at 1,000 rpm for 10 minutes. After 10 minutes of reaction time, samples may be drawn and immediately quenched by adding the same volume of 1.4% hydrochloric acid. After mixing of the sample, cells may be removed by centrifugation for 1 minute at 10,000 rpm and the amount of acrylamide formed is determined by analyzing the clear supernatant by HPLC, as described in Example 4. For affirmation of a biocatalyst to be capable of converting acrylonitrile to acrylamide in context with the present invention, the concentration of acrylamide shall be between 0.25 and 1.25 mmol/l - if necessary, the sample has to be diluted accordingly and the bioconversion has to be repeated. The activity may then be deduced from the concentration of acrylamide by dividing the acrylamide concentration derived from HPLC analysis by the reaction time, which has been 10 minutes and by multiplying this value with the dilution factor between HPLC sample and original sample. Activities >5 U/mg dry cell weight, preferably >25 U/mg dry cell weight, more preferably >50 U/mg dry cell weight, most preferably >100 U/mg dry cell weight indicate the presence of a functional biocatalyst and are considered as biocatalyst capable of converting acrylonitrile to acrylamide in context with the present invention.

In addition, in any one of the methods described and provided herein, water is added to the reactor. The water may be added as such, be part of the biocatalyst as described herein, be part of an acrylonitrile solution as described herein, or otherwise be added. In case that the water is added as such, in general tap water or deionized water may be used. The water may also be part of an aqueous composition, such as an aqueous solution of a salt. In particular, a buffer may be employed.

In accordance with any one of the methods of the present invention, the biocatalyst capable of converting acrylonitrile to acrylamide may be a microorganism which encodes the enzyme nitrile hydratase (NHase). With this regard, it is not relevant for the present invention whether the microorganism is naturally encoding nitrile hydratase, or whether it has been genetically modified to encode said enzyme, or whether a microorganism naturally encoding nitrile hydratase has been modified such as to be able to produce more and/or enhanced nitrile hydratase. As used herein, the expression "biocatalyst (e.g., microorganism) encoding (the enzyme) nitrile hydratase" or the like generally means that such a microorganism is generally also able to produce and stably maintain nitrile hydratase. That is, as used herein and as readily understood by the skilled person, a biocatalyst (e.g., a microorganism) to be employed in accordance with the present invention which (naturally or non-naturally) encodes nitrile hydratase is generally also capable of producing and stably maintaining nitrile hydratase. However, in accordance with the present invention, it is also possible that such microorganisms only produced nitrile hydratase during cultivation (or fermentation) of the microorganism - thus then containing nitrile hydratase - before being added to a reactor according to step (a) of any one of the methods described and provided herein. In such a case, it is possible that the microorganisms do not produce nitrile hydratase during the methods described and provided herein any more, but they act only via the nitrile hydratase units which they have produced before and which they still contain. As readily understood by the person skilled in the art, it is also possible that some nitrile hydratase molecules may leave the microorganism (e.g., due to lysis of the microorganism) and act freely in the solution as biocatalyst. As such, it also possible that the term "biocatalyst" as used herein encompasses the enzyme nitrile hydratase per se, as long as it is able to convert acrylonitrile to acrylamide as described and exemplified herein. In context with the present invention, it is also possible to directly employ nitrile hydratase as biocatalyst.

In context with the present invention, microorganisms naturally encoding nitrile hydratase, which can be used as biocatalyst in any one of the methods described herein, comprise species belonging to a genus selected from the group consisting of *Rhodococcus, Aspergillus, Acidovorax, Agrobacterium, Bacillus, Bradyrhizobium, Burkholderia, Escherichia, Geobacillus, Klebsiella, Mesorhizobium, Moraxella, Pantoea, Pseudomonas, Rhizobium, Rhodopseudomonas, Serratia, Amycolatopsis, Arthrobacter, Brevibacterium, Corynebacterium, Microbacterium, Micrococcus, Nocardia, Pseudonocardia, Trichoderma, Myrothecium, Aureobasidium, Candida, Cryptococcus, Debaryomyces, Geotrichum, Hanseniaspora, Kluyveromyces, Pichia, Rhodotorula, Comomonas,* and *Pyrococcus.* In preferred embodiments of the invention the biocatalyst is selected from bacteria of the genus *Rhodococcus, Pseudomonas, Escherichia* and *Geobacillus.*

Preferred biocatalysts to be employed in context with any one of the methods of the present invention comprise representatives of the genus *Rhodococcus.* Species suitable as biocatalyst to be employed in context with any one of the methods of the present invention may comprise, e.g., *Rhodococcus rhodochrous* (e.g., NCIMB 41164, J1/FERM-BP 1478, M33 or M8), *Rhodococcus pyridinovorans, Rhodococcus erythropolis, Rhodococcus equi, Rhodococcus ruber, Rhodococcus opacus, Aspergillus niger, Acidovorax avenae, Acidovorax facilis, Agrobacterium tumefaciens, Agrobacterium radiobacter, Bacillus subtilis, Bacillus pallidus, Bacillus smithii, Bacillus sp BR449, Bradyrhizobium oligotrophicum, Bradyrhizobium diazoefficiens, Bradyrhizobium japonicum, Burkholderia cenocepacia, Burkholderia gladioli, Escherichia coli, Geobacillus sp. RAPc8, Klebsiella oxytoca, Klebsiella pneumonia, Klebsiella variicola, Mesorhizobium ciceri, Mesorhizobium opportunistum, Mesorhizobium sp F28, Moraxella, Pantoea endophytica, Pantoea agglomerans, Pseudomonas chlororaphis, Pseudomonas putida, Rhizobium, Rhodopseudomonas palustris, Serratia liquefaciens, Serratia marcescens, Amycolatopsis, Arthrobacter, Brevibacterium sp CH1, Brevibacterium sp CH2, Brevibacterium sp R312, Brevibacterium imperiale, Brevibacterium casei, Corynebacterium nitrilophilus, Corynebacterium pseudodiphteriticum, Corynebacterium glutamicum, Corynebacterium hoffmanii, Microbacterium imperiale, Microbacterium smegmatis, Micrococcus luteus, Nocardia globerula, Nocardia rhodochrous, Nocardia sp 163, Pseudonocardia thermophila, Trichoderma, Myrothecium verrucaria, Aureobasidium pullulans, Candida famata, Candida guilliermondii, Candida tropicalis, Cryptococcus flavus, Cryptococcus sp UFMG- Y28, Debaryomyces hanseii, Geotrichum candidum, Geotrichum sp JR1, Hanseniaspora, Kluyveromyces thermotolerans, Pichia kluyveri, Rhodotorula glutinis, Comomonas testosteroni, Pyrococcus abyssi, Pyrococcus furiosus,* or *Pyrococcus horikoshii.*

According to an embodiment of any one of the methods of the present invention, the biocatalyst to be employed belongs to the species *Rhodococcus rhodochrous.* Particular examples for strains belonging to *Rhodococcus rhodochrous* which may be employed in context with any one of the methods described herein comprise NCIMB 41164, J1 (FERM-BP 1478), M33 and M8.

Alternatively or in addition to *Rhodococcus rhodochrous,* the biocatalyst employed in any one of the methods described herein may be *Rhodococcus pyridinovorans.*

According to the present invention, combinations of these microorganisms can be used as well. Further, the above microorganisms can be cultured by any method that is appropriate for a given microbial species. The microbial biocatalyst of the present invention that is prepared from microorganisms refers to a culture solution obtained by culturing microorganisms, cells obtained by a harvesting process or the like, cell disrupted by ultrasonication or the like, or those prepared after cell disruption including a crude enzyme, a partially-purified enzyme or a purified enzyme. A mode to use the microbial catalyst may be appropriately selected depending on enzyme stability, production scale and the like.

In some embodiments of the present invention, the biocatalyst used for converting acrylonitrile to acrylamide as described herein may be washed before the use in said reaction. In some embodiments, the biocatalyst may be washed once with water, a buffer or the like, and then washed with acrylic acid before the reaction. In some embodiments the biocatalyst used herein is washed with acrylic acid before the reaction as described in detail in EP1380652. In some embodiments the biocatalyst may be washed with acrylic acid immediately before the reaction. Further, any washing methods can be employed. Examples of such a method that can be applied according to the present invention include a method which involves repeated washing and centrifugation, and a washing method using a hollow fiber membrane. Further, immobilized biocatalysts can be washed by repeating agitation and precipitation of the immobilized catalysts in a wash and the removal of supernatant. Any washing method and any number of washing can be appropriately set in consideration of washing efficiency, enzyme stability and the like. The concentration of acrylic acid to be used for washing is preferably between 0.01 % by mass and 10 % by mass in an aqueous acrylic solution. More preferably, the concentration is between 0.05 % by mass and 1 % by mass, and most preferably is 0.1 % by mass.

In context with the present invention, nitrile hydratase encoding microorganisms which are not naturally encoding nitrile hydratase may be genetically engineered microorganisms which naturally do not contain a gene encoding a nitrile hydratase but which have been manipulated such as to contain a polynucleotide encoding a nitrile hydratase (e.g., via transformation, transduction, transfection, conjugation, or other methods suitable to transfer or insert a polynucleotide into a cell as known in the art; cf. Sambrook and Russell 2001, Molecular Cloning: A Laboratory Manual, CSH Press, Cold Spring Harbor, NY, USA), thus enabling the microorganisms to produce and stably maintain the nitrile hydratase enzyme. For this purpose, it may further be required to insert additional polynucleotides which may be necessary to allow transcription and translation of the nitrile hydratase gene or mRNA, respectively. Such additional polynucleotides may comprise, inter alia, promoter sequences, polyT- or polyU-tails, or replication origins or other plasmid-control sequences. In this context, such genetically engineered microorganisms which naturally do not contain a gene encoding a nitrile hydratase but which have been manipulated such as to contain a polynucleotides encoding a nitrile hydratase may be prokaryotic or eukaryotic microorganisms. Examples for such prokaryotic microorganisms include, e.g., representatives of the species Escherichia coli. Examples for such eukaryotic microorganisms include, e.g., yeast (e.g., *Saccharomyces cerevisiae*)*.*

In context of the present invention, the term "nitrile hydratase" (also referred to herein as NHase) generally means an enzyme which is capable of catalyzing the bioconversion (i.e. hydration) of acrylonitrile to acrylamide. Such an enzyme may be, e.g., the enzyme registered under IUBMB nomenclature as of September 30, 2014: EC 4.2.1.84; CAS-No. 2391-37-5. However, the term "nitrile hydratase" as used herein also encompasses modified or enhanced enzymes which are e.g. capable of converting acrylonitrile to acrylamide more quickly, or which can be produced at a higher yield/time-ratio, or which are more stable, as long as they are capable to catalyze bioconversion (i.e. hydration) of acrylonitrile to acrylamide. Methods for determining the ability of a given biocatalyst (e.g., microorganism or enzyme) for catalyzing the bioconversion of acrylonitrile to acrylamide are known in the art. A method for the determination of the activity of a given biocatalyst to act as a nitrile hydratase in the sense of the present invention is described elsewhere herein.

In context with the present invention, the nitrile hydratase may be a polypeptide encoded by a polynucleotide which comprises or consists of a nucleotide sequence which is at least 70%, preferably at least 75%, more preferably at least 80%, more preferably at least 85%, more preferably at least 90%, more preferably at least 95%, more preferably at least 96%, more preferably at least 97%, more preferably at least 98%, more preferably at least 99%, more preferably at least 99,5%, and most preferably 100% identical to the nucleotide sequence of SEQ ID NO: 1 (alpha-subunit of nitrile hydratase of *R. rhodochrous*: gtgagcgagcacgtcaataagtacacggagtacgaggcacgtaccaaggcgatcgaaaccttgctgtacgagcgagg gctcatcacgcccgccgcggtcgaccgagtcgtttcgtactacgagaacgagatcggcccgatgggcggtgccaaggtc gtggccaagtcctgggtggaccctgagtaccgcaagtggctcgaagaggacgcgacggccgcgatggcgtcattgggc tatgccggtgagcaggcacaccaaatttcggcggtcttcaacgactcccaaacgcatcacgtggtggtgtgcactctgtgtt cgtgctatccgtggccggtgcttggtctcccgcccgcctggtacaagagcatggagtaccggtcccgagtggtagcggac cctcgtggagtgctcaagcgcgatttcggtttcgacatccccgatgaggtggaggtcagggtttgggacagcagctccgaa atccgctacatcgtcatcccggaacggccggccggcaccgacggttggtccgaggaggagctgacgaagctggtgagc cgggactcgatgatcggtgtcagtaatgcgctcacaccgcaggaagtgatcgtatga) and/or to the nucleotide sequence of SEQ ID NO: 3 (beta-subunit of nitrile hydratase of R. rhodochrous: atggatggtatccacgacacaggcggcatgaccggatacggaccggtcccctatcagaaggacgagcccttcttccact acgagtgggagggtcggaccctgtcaattctgacttggatgcatctcaagggcatatcgtggtgggacaagtcgcggttctt ccgggagtcgatggggaacgaaaactacgtcaacgagattcgcaactcgtactacacccactggctgagtgcggcaga acgtatcctcgtcgccgacaagatcatcaccgaagaagagcgaaagcaccgtgtgcaagagatccttgagggtcggta cacggacaggaagccgtcgcggaagttcgatccggcccagatcgagaaggcgatcgaacggcttcacgagccccact ccctagcgcttccaggagcggagccgagtttctctctcggtgacaagatcaaagtgaagagtatgaacccgctgggaca cacacggtgcccgaaatatgtgcggaacaagatcggggaaatcgtcgcctaccacggctgccagatctatcccgagag cagctccgccggcctcggcgacgatcctcgcccgctctacacggtcgcgttttccgcccaggaactgtggggcgacgac ggaaacgggaaagacgtagtgtgcgtcgatctctgggaaccgtacctgatctctgcgtga), provided that the polypeptide encoded by said polynucleotide is capable of catalyzing hydration of acrylonitrile to acrylamide (i.e. has nitrile hydratase activity) as described and exemplified herein. Also in the context with the present invention, the nitrile hydratase may be a polypeptide which comprises or consists of an amino acid sequence which is at least 70%, preferably at least 75%, more preferably at least 80%, more preferably at least 85%, more preferably at least 90%, more preferably at least 95%, more preferably at least 96%, more preferably at least 97%, more preferably at least 98%, more preferably at least 99%, more preferably at least 99,5%, and most preferably 100% identical to the amino acid sequence of SEQ ID NO: 2 (alpha-subunit of nitrile hydratase of R. rhodochrous: vsehvnkyte yeartkaiet IIyerglitp aavdrvvsyy eneigpmgga kvvakswvdp eyrkwleeda taamaslgya geqahqisav fndsqthhvv vctlcscypw pvlglppawy ksmeyrsrvv adprgvlkrd fgfdipdeve vrvwdsssei ryiviperpa gtdgwseeel tklvsrdsmi gvsnaltpqe viv) and/or to the amino acid sequence of SEQ ID NO: 4 (beta-subunit of nitrile hydratase of R. rhodochrous: mdgihdtggm tgygpvpyqk depffhyewe grtlsiltwm hlkgiswwdk Srffresmgn enyvneirnsy ythwlsaae rilvadkiit eeerkhrvqe ilegrytdrk psrkfdpaqi ekaierlhep hslalpgaep sfslgdkikv ksmnplghtr cpkyvrnkig eivayhgcqi ypesssaglg ddprplytva fsaqelwgdd gngkdvvcvd Iwepylisa), provided that said polypeptide is capable of catalyzing hydration of acrylonitrile to acrylamide as described and exemplified herein.

The level of identity between two or more sequences (e.g., nucleic acid sequences or amino acid sequences) can be easily determined by methods known in the art, e.g., by BLAST analysis. Generally, in context with the present invention, if two sequences (e.g., polynucleotide sequences or amino acid sequences) to be compared by, e.g., sequence comparisons differ in identity, then the term "identity" may refer to the shorter sequence and that part of the longer sequence that matches said shorter sequence. Therefore, when the sequences which are compared do not have the same length, the degree of identity may preferably either refer to the percentage of nucleotide residues in the shorter sequence which are identical to nucleotide residues in the longer sequence or to the percentage of nucleotides in the longer sequence which are identical to nucleotide sequence in the shorter sequence. In this context, the skilled person is readily in the position to determine that part of a longer sequence that matches the shorter sequence. Furthermore, as used herein, identity levels of nucleic acid sequences or amino acid sequences may refer to the entire length of the respective sequence and is preferably assessed pair-wise, wherein each gap is to be counted as one mismatch. These definitions for sequence comparisons (e.g., establishment of "identity" values) are to be applied for all sequences described and disclosed herein.

Moreover, the term "identity" as used herein means that there is a functional and/or structural equivalence between the corresponding sequences. Nucleic acid/amino acid sequences having the given identity levels to the herein-described particular nucleic acid/amino acid sequences may represent derivatives/variants of these sequences which, preferably, have the same biological function. They may be either naturally occurring variations, for instance sequences from other varieties, species, etc., or mutations, and said mutations may have formed naturally or may have been produced by deliberate mutagenesis. Furthermore, the variations may be synthetically produced sequences. The variants may be naturally occurring variants or synthetically produced variants or variants produced by recombinant DNA techniques. Deviations from the above-described nucleic acid sequences may have been produced, e.g., by deletion, substitution, addition, insertion and/or recombination. The term "addition" refers to adding at least one nucleic acid residue/amino acid to the end of the given sequence, whereas "insertion" refers to inserting at least one nucleic acid residue/amino acid within a given sequence. The term "deletion" refers to deleting or removal of at least one nucleic acid residue or amino acid residue in a given sequence. The term "substitution" refers to the replacement of at least one nucleic acid residue/amino acid residue in a given sequence. Again, these definitions as used here apply, mutatis mutandis, for all sequences provided and described herein.

Generally, as used herein, the terms "polynucleotide" and "nucleic acid" or "nucleic acid molecule" are to be construed synonymously. Generally, nucleic acid molecules may comprise inter alia DNA molecules, RNA molecules, oligonucleotide thiophosphates, substituted ribo-oligonucleotides or PNA molecules. Furthermore, the term "nucleic acid molecule" may refer to DNA or RNA or hybrids thereof or any modification thereof that is known in the art (see, e.g., US 5525711, US 471 1955, US 5792608 or EP 302175 for examples of modifications). The polynucleotide sequence may be single- or doublestranded, linear or circular, natural or synthetic, and without any size limitation. For instance, the polynucleotide sequence may be genomic DNA, cDNA, mitochondrial DNA, mRNA, antisense RNA, ribozymal RNA or a DNA encoding such RNAs or chimeroplasts (Gamper, Nucleic Acids Research, 2000, 28, 4332 - 4339). Said polynucleotide sequence may be in the form of a vector, plasmid or of viral DNA or RNA. Also described herein are nucleic acid molecules which are complementary to the nucleic acid molecules described above and nucleic acid molecules which are able to hybridize to nucleic acid molecules described herein. A nucleic acid molecule described herein may also be a fragment of the nucleic acid molecules in context of the present invention. Particularly, such a fragment is a functional fragment. Examples for such functional fragments are nucleic acid molecules which can serve as primers.

The reaction time of the bioconversion of acrylonitrile to the acrylamide is not specifically restricted either provided that the amide reaction solution of a desired concentration is obtained. Although the reaction time depends upon the amount of the catalyst used and the conditions such as temperature, it is specifically in the range of usually 1 to 80 hours, preferably 2 to 40 hours, based on one reactor. Although the bioconversion of the nitrile compound to the amide compound is usually carried out at atmospheric pressure, it may be carried out under pressure in order to increase solubility of the nitrile compound in the aqueous medium. The reaction temperature is not specifically restricted provided that it is not lower than the ice point of the aqueous medium. However, it is desirable to carry out the bioconversion at a temperature of usually 0 to 50°C, preferably 10 to 40°C, more preferably 20 to 37°C., even more preferably 15 to 30°C. The bioconversion of the nitrile compound to the amide compound may be carried out by any of batch process, fed-batch process or continuous process, and the bioconversion may be carried out by selecting its reaction system from reaction systems such as suspended bed, a fixed bed, a fluidized bed and the like or by combining different reaction systems according to the form of the catalyst.

According to any one of the methods described herein, acrylonitrile is added to the reactor. With this respect, the acrylonitrile may be added continuously or intermittently. Addition of acrylonitrile may be at constant or variable feed rate or batch-wise. The acrylonitrile may be added in pure form or in solution. For example, an aqueous solution of acrylonitrile may be used. Typically, the reaction is carried out in fed-batch mode wherein acrylonitrile is added to the reaction solution intermittently or continuously. Preferably acrylonitrile is added continuously. The term "continuously" refers to the continuous feed of acrylonitrile for at least 15 min, at least 30 min, at least 60 min, at least 90 min, at least 120 min, at least 240 min or at least 360 min. The term "intermittently" refers to the addition of acrylonitrile in a non-continuous manner, i.e. wherein there is a break between at least two additions of acrylonitrile.

In some embodiments, acrylonitrile is added to the reaction mixture independently of the acrylonitrile concentration in the reaction mixture. The addition independently of the acrylonitrile concentration may occur at constant feed rate, variable feed rate or batch-wise. This means that the feed of the acrylonitrile concentration is not controlled so that a specific content of acrylonitrile is maintained during the bioconversion. For example acrylonitrile is added to the reaction at a constant rate, e.g. at a constant rate of 95 to 170 g / kg reaction solution / hour.

Any one of the methods described herein may be carried out using a continuous process. In particular, the term "continuous process" as used herein refers to a method, wherein an aqueous acrylamide solution is produced in a continuous manner without collecting the entire reaction mixture in the reactor. This means that the raw materials for the reaction, which may comprise the biocatalyst, water and acrylonitrile, are fed to the reactor continuously or intermittently and that the obtained product is recovered from the reactor continuously or intermittently.

Alternatively, any one of the methods of the present invention may be carried out using a fed-batch process. In particular, the terms "fed-batch process" or "fed-batch mode" as used herein may comprise that an aqueous acrylamide solution is produced in a discontinuous manner. According to a non-limiting example for carrying out such a semi-batch process water, a certain amount of acrylonitrile and the biocatalyst are placed in a reactor. Further acrylonitrile is then added during the bioconversion until a desired content of acrylamide of the reaction mixture is reached. After such desired content of acrylamide is reached, the obtained reaction mixture is entirely recovered from the reactor, before new reactants are placed therein.

When adding the biocatalyst to the reactor in any one of the methods of the present invention, the biocatalyst may be taken directly from the fermentation broth. It is further envisaged that the biocatalyst may be employed in the form of a fermentation broth in the methods disclosed herein. Thus, the biocatalyst does not need to be isolated from the fermentation broth, and a fermentation broth comprising the biocatalyst may be used for the bioconversion. For example, a fermentation broth comprising the biocatalyst may be added to the reactor in step (a) of the methods of the present invention. Alternatively, in accordance with any one of the methods described herein, the biocatalyst may have been dried before being added to the reactor. In this context the term "before" does not necessarily mean that the biocatalyst has been dried and is then directly added to the reactor. It is rather sufficient that the biocatalyst has undergone a drying step at any time before it is added to the reactor, independently of whether further steps between the drying and the addition are performed or not. As non-limiting examples, such further steps between the drying step and the addition to the reactor may be storage or reconstitution. However, it is also possible to add the biocatalyst to the reactor directly after drying.

A reconstituted biocatalyst is a dried biocatalyst that is suspended, i.e. present in a slurry or dissolved in an aqueous solution such as water or a buffer solution having a physiologic pH, or aqueous composition. The latter may contain one or more further ingredients such as glucose. Reconstitution refers herein to the addition of an aqueous composition to the dried microorganism before the microorganism is contacted with the nitrile compound. Accordingly, in any one of the methods described herein, a biocatalyst which is treated by a drying step and then is suspended in an aqueous composition may be added to the reaction mixture. Such aqueous compositions include, without limitation, water (e.g. deionized water), and a buffer (e.g. phosphate buffer).

The inventors have surprisingly found that by using a biocatalyst, which has undergone a drying step, the concentration of acrylic acid of the reaction mixture at the end of the reaction obtained by any one of the methods described herein is further reduced in comparison to the case that a biocatalyst is used which has not undergone drying before being employed in the bioconversion.

Regarding the drying method, in any one of the methods described an provided herein, a biocatalyst may be used which has been dried using freeze-drying, spray drying, heat drying, vacuum drying, fluidized bed drying and/or spray granulation. With this respect, spray drying and freeze drying are preferred, since in general by using a biocatalyst, which has been subjected to spray- or freeze drying, a higher reduction of the acrylic acid concentration in the obtained reaction mixture at the end of the reaction is achieved compared to employing a biocatalyst which has been dried using other methods.

According to any one of the methods of the present invention a dried biocatalyst may be added to the reactor. This means that the biocatalyst is added to the reactor in a dried form. In particular, the biocatalyst may have the form of a powder or a granule. As an alternative to adding a dried biocatalyst to the reactor, the dried biocatalyst may be reconstituted before being added to the reactor. For example, the biocatalyst may be reconstituted by suspending in an aqueous composition. With this respect, the aqueous composition may be water or a buffer. As a further alternative, a biocatalyst in form of a matrix bound microorganism may be added to the reactor.

The term "dried biocatalyst" as used herein refers to a biocatalyst that has been subjected to a drying step. A dried biocatalyst typically has a moisture content of less than about 20 w/w %, more preferably less than about 15 w/w %, even more preferably less than about 14 w/w %, most preferably from about 5 to about 10 w/w % based on the total weight of the biocatalyst sample. Methods of determining the moisture content are familiar to the skilled person. For example, in the context of the present invention the moisture content of a sample of the dried biocatalyst may be determined via thermogravimetric analysis. At the beginning of the thermogravimetric analysis the initial weight of the sample is determined. The sample is then heated and the moisture vaporizes. Heating is continued until the sample weight remains constant. The difference between the constant weight at the end of the analysis and the initial weight represents the amount of water vaporized during the analysis, which allows for calculation of the moisture content of the sample. For determination of the moisture content via thermogravimetric analysis, the biocatalyst sample may be, for example, analyzed on a 'Mettler Toledo HB43-S Halogen moisture analyzer', operated at 130 °C until the sample weight remains constant for at least 30 seconds.

By performing any one of the methods described herein the aqueous acrylamide solution may be obtained along with the biocatalyst. Accordingly, the biocatalyst may be separated from the obtained aqueous acrylamide solution. Such a separation of the biocatalyst may be performed with regard to the desired applications, which may, for example, include the homopolymerization or copolymerization of the acrylamide. Suitable methods for separation of the biocatalyst are known in the art and include, for example, centrifugation, sedimentation (e.g., with flocculation), membrane separation and filtration.

Without being bound by theory, it is believed that drying of the biocatalyst (i.e. microorganism) decreases the activity of Amidase, whereby the NHase activity is thought to decrease to a lower extent or remains unchanged. In fact, the present inventors observed that the activity of NHase was higher than the activity of Amidase, when the NHase and Amidase producing microorganism was pre-treated by a drying step before being contacted with a nitrile compound that should be subject to bioconversion (nitrile compound into amide compound) by said microorganism.

The biocatalyst may be added to the reaction mixture in the form of a powder, granule, and/or suspension. It is also possible to use a matrix-bound biocatalyst.

As used herein, the term "enzyme unit (U)" refers to the amount of enzyme that produces 1 µmole of product per minute under specified assay conditions, i.e. at 25° C. For example, with regard to NHase the term "enzyme unit (U)" is defined as the amount of enzyme required to produce 1 µmole of acrylamide per minute from acrylonitrile under assay conditions of 25° C in a phosphate buffer, pH 7,0.

In other embodiments a saccharide is added to the reaction mixture.

The term "saccharide" that is used herein refers in essence to all kinds of saccharide(s) that can be detected and/or quantified by the phenol sulfuric acid method. The Phenol - Sulphuric Acid Method is a well-established method used in measuring saccharides as such as well as sugar content in oligosaccharides, proteoglycans, glycoproteins and glycolipids etc ("Colorimetric Method for Determination of Sugars and Related Substances" in Anal. Chem., 1956, 28 (3), pp 350-356. This method is hereby incorporated by reference to said publication.) Several manufactures offer standardized Assay kits, which are all based on the Phenol Sulphuric Acid method (for example BioVisions Inc., San Francisco, US; distributes "Total Carbohydrate Colorimetric Assay Kit"). Examples of the "saccharides" include monosaccharides, oligosaccharides, polysaccharides, and mixtures thereof. "Polysaccharides" contain more than ten monosaccharide units which are connected via glycosidic bonds and which are either linear or branched. "Polysaccharide" therefore includes but is not limited to e.g. starch, cellulose, mannan, galactomannan, callose, glycogen, chitin, pectin, arabinoxylans, bacterial capsular polysaccharides, etc. to name some; these polysaccharides are preferably water soluble. "Oligosaccharides" means up to ten (including ten) monosaccharides which are connected via glycosidic bonds and which are either linear or branched - oligosaccharides of the invention are preferably water soluble. Preferred oligosaccharides are sucrose, lactose and maltose. "Monosaccharides" refers to the well-known ketoses or aldoses that can be classified, based on the number of C-atoms, as dioses, trioses, teroses, pentoses, hexoses, heptoses, octoses and so on. Well-known variations of these monosaccharides are also contemplated (such as e.g. amino sugars of the monosaccharides such as galactosamine, glucosamine, sialic acid etc. or sulfosugars such as sulfoquinovose). Glucose, mannose, fructose, arabinose, furanose, pyranose, xylose, lyxose, ribose, allose, altrose, gulose, iodose, galactose, talose, etc. are preferred saccharides. Glucose is a particularly preferred saccharide in the context of the present invention.
"Saccharides" further includes glycoconjugates, such as glycoproteins and glycolipids etc. Glucose is a particularly preferred saccharide to be added to or admixed with the reaction mixture.

Usually the methods according to the invention comprise the following steps:
(a) preparing a reaction mixture to obtain a reaction mixture for bioconversion comprising:
   (i) a biocatalyst capable of converting acrylonitrile to acrylamide;
   (ii) acrylonitrile;
   (iii) water; and
(b) performing a bioconversion on the reaction mixture obtained in step (a);

In some embodiments the biocatalyst may be contacted with urea.

The method of contacting the biocatalyst with urea is not particularly limited. Thus, in case that the contacting of the biocatalyst with urea is not further specified, any method known in the art may be used, which is suitable for contacting the biocatalyst with urea. In context of the present invention the term "contacting" the biocatalyst with urea must not be construed as limiting the methods and uses described and provided herein to cases where urea is actively contacted as such with the biocatalyst. That is, the term "contacting" in this context also comprises cases where urea is brought into contact with the biocatalyst together with other components, e.g. together with water.

It is preferred that the contacting of the biocatalyst with urea is carried out before the biocatalyst is employed in the bioconversion of acrylonitrile to acrylamide, i.e. before the biocatalyst is contacted with the acrylonitrile. With this respect, contacting of the biocatalyst with urea may e.g. be performed outside of the reactor. The biocatalyst obtained by the contacting with urea is then added to the reactor and combined with acrylonitrile and water. Alternatively, the contacting of the biocatalyst with urea may be performed inside of the reactor. After the contacting of the biocatalyst with urea has been carried out inside of the reactor, the biocatalyst obtained is then combined with acrylonitrile and water.

In any one of the methods described herein above and below the contacting of the biocatalyst with urea may comprise contacting the biocatalyst with urea after cultivation. For example, after cultivation a biocatalyst, which optionally may have been dried, may be used and brought intentionally into contact with urea. Thus, in case that the biocatalyst is contacted with urea after cultivation, the contacting may be carried out as an active step. "Active step" in particular means that the biocatalyst and urea are brought into contact intentionally. As a non-limiting example, in any one of the methods described herein above and below, the biocatalyst may be contacted with urea by suspending the biocatalyst with a solution of urea. For this purpose, in general any solvent which is suitable to dissolve urea can be used. Preferably, water is used as the solvent such that the biocatalyst is suspended with an aqueous solution of urea. According to another non-limiting example for contacting the biocatalyst with urea, in any one of the methods described herein solid urea may be added to a suspension or solution of the biocatalyst, e.g. a suspension of the biocatalyst in water. As used with respect to any one of the methods described herein above and below, the term "cultivation" denotes a process, wherein the biocatalyst, which may be, for example, a microorganism, is grown, preferably in a(n) (aqueous) medium. For example, the cultivation as used herein comprises addition of oxygen (e.g., by aerating the cultivation medium with air) and may preferably further comprise the presence of C- and N-sources in the medium. Such cultivation is generally carried out under conditions wherein the biocatalyst is present in a fermentation broth comprising a culture medium. The specific culture medium and conditions of cultivation are not particularly limited, and any suitable method, culture medium and/or condition known for the cultivation of a biocatalyst may be used. As used with respect to any one of the methods described herein above and below, the term "after cultivation" in particular refers to the situation that the biocatalyst has been separated from the fermentation broth by any method suitable therefore, such as e.g. filtration and/or centrifugation. The term "separated" does not necessarily denote a complete separation of the biocatalyst from the culture medium. Thus, the biocatalyst may comprise residual parts of the fermentation broth and/or the culture medium even after separation.

Alternatively or in addition to contacting the biocatalyst with urea after cultivation, in any one of the methods described herein above and below, the contacting of the biocatalyst with urea may comprise contacting the biocatalyst with urea during cultivation. "During cultivation" in particular means that a contact between the biocatalyst and the urea is provided within the fermentation broth used in the cultivation. With this respect, certain amounts of urea may be present in the fermentation broth during cultivation. It is thus possible, that certain amounts of urea are contained in a fermentation broth containing the biocatalyst which is then transferred into the reactor. Preferably, in case that the biocatalyst is taken from a fermentation broth containing urea, the biocatalyst is not washed, such that residual amounts of urea may be transferred to the reactor. Also, residues of urea may still be connected to dried biocatalyst which may be added to the reactor. As a non-limiting option, in case that the biocatalyst is contacted with urea during cultivation, the biocatalyst may be further contacted with urea after cultivation. In particular, after cultivation the biocatalyst may be contacted with urea actively, which may be e.g. carried out by suspending the biocatalyst with a solution of urea or by adding solid urea to a suspension or solution of the biocatalyst as non-limiting examples.

The skilled person understands that present application also contemplates methods according to the invention, (i.e. for example defined by the addition of the biocatalyst of less than 200 U/g acrylonitrile) in which a saccharide is added to the reaction mixture and wherein the biocatalyst is contacted with urea.

A further aspect refers to a method for producing an acrylamide homopolymer or copolymer comprising the method for producing acrylamide from acrylonitrile in an aqueous solution as described herein and further comprising the step of polymerizing the acrylamide obtained by the method for producing acrylamide from acrylonitrile in an aqueous solution as described herein.

With this respect, in case of a homopolymer the term "polymerizing" refers to a homopolymerization reaction, while in case of a copolymer the term "polymerizing" refers to a copolymerization reaction. The homopolymerization or copolymerization may be performed using an aqueous acrylamide solution obtainable or being obtained by any one of the methods described herein. In particular, an aqueous acrylamide solution may be used, from which the biocatalyst has been separated prior to the polymerization. Alternatively, the acrylamide may have been isolated from the aqueous acrylamide solution before being subjected to homopolymerization or copolymerization.

Acrylic acid is an undesired byproduct in the bioconversion of acrylonitrile to acrylamide as it may have a negative impact on downstream processing of acrylamide, e.g. on the production of acrylamide polymers. Since the present invention allows for preparation of aqueous acrylamide solutions having a reduced concentration of acrylic acid, in case that such aqueous acrylamide solutions are used for homopolymerization or copolymerization reactions, also the obtained acrylamide homopolymers or copolymers have a reduced content of acrylic acid. Such acrylamide homopolymers or copolymers exhibit improved physical properties, such as solubility, and performance.

High acrylic acid contents within acrylamide solutions can lead to reduced performance of the resulting polyacrylamide homopolymers and copolymers, especially for cationic polyacrylamide products, i.e. copolymers of acrylamide with cationic co-monomers. This is highly evident for cationic copolymers with low cationic co-monomer contents. Without wishing to be bound by any theory, molar equivalent amounts of anionic acrylic acid and the cationic co-monomers within the copolymer chain results in the generation of charge complexes. This can significantly impair the physical properties of the polyacrylamide material, reducing solubility and performance in applications such as water treatment, paper making, oil recovery or mining.

Regarding this impact of acrylic acid, the acrylamide homopolymer or copolymer described and provided herein is preferably a cationic polyacrylamide. As generally known to a person skilled in the art, the term "cationic polyacrylamide" denotes a copolymer which in addition to acrylamide monomers contains cationic co-monomers, such as, e.g., co-monomers which comprise quaternary ammonium groups. Particularly preferred is a cationic polyacrylamide having an acrylic acid content of 60,000 ppm or less, preferably of 20,000 ppm or less, more preferably of 10,000 ppm or less, even more preferably of 2,000 ppm or less, and most preferably of 1,500 ppm or less, wherein the indications of ppm each relate to weight parts and are each referred to the total weight of the solid acrylamide homopolymer or copolymer.

In general, the acrylic acid content of any polymer or copolymer described herein may be determined using methods known in the art, e.g., NMR spectroscopy as described in European Polymer Journal (2007), 43(3): 824-834 or preferably the HPLC method described in Example 4.

Acrylamide homopolymers and/or copolymers are, for example, used in oilfield applications. In particular, use of acrylamide homopolymers and/or copolymers is made in tertiary oil recovery, which is also denoted as enhanced oil recovery. With this respect, in methods of tertiary oil recovery an aqueous solution of the polymer may be injected into the rock in order to promote oil displacement and thus increase the yield of crude oil. The present invention is therefore also related to an aqueous solution of any acrylamide homopolymer and/or copolymer described herein. As the water for the aqueous solution seawater, such as artificial sea water may be used.

The term "artificial sea water" refers to a solution prepared by the standard ASTM D1141-98, i.e. NaCl 24.53 g/l, MgCl2 5.20 g/l, Na2SO4 4.09 g/l, CaCl2 1.16 g/l, KCl 0.695 g/l, NaHCO3 0.201 g/l, KBr 0.101 g/l, H3BO3 0.027 g/l, SrCl2 0.025 g/l, NaF 0.003 g/l.

Another aspect of the invention refers to an acrylamide homopolymer or copolymer obtainable by the method for producing an acrylamide homopolymer or copolymer as described herein.

Another aspect refers to an acrylamide homopolymer or copolymer, in particular to an acrylamide homopolymer or copolymer obtainable by the method for producing an acrylamide homopolymer or copolymer as described herein, having an acrylic acid content of 60,000 ppm or less, preferably of 20,000 ppm or less, more preferably of 10,000 ppm or less, even more preferably of 2,000 ppm or less, and most preferably of 1,500 ppm or less, wherein the indications of ppm each relate to weight parts and are each referred to the total weight of the solid acrylamide homopolymer or copolymer..

Unless otherwise required by context, singular terms shall include pluralities and plural terms shall include the singular. As used herein and in the appended claims, the singular forms "a", "an", and "the", include plural referents unless the context clearly indicates otherwise. Thus, for example, reference to "a reagent" includes one or more of such different reagents, and reference to "the method" includes reference to equivalent steps and methods known to those of ordinary skill in the art that could be modified or substituted for the methods described herein.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or" as used herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or sometimes when used herein with the term "having". For the purposes of the present invention, the term "consisting of" is considered to be a preferred embodiment of the term "comprising of". If hereinafter a group is defined to comprise at least a certain number of embodiments, this is also to be understood to disclose a group which preferably consists only of these embodiments.

The terms "about" or "approximately" in the context of the present invention denote an interval of accuracy that the person skilled in the art will understand to still ensure the technical effect of the feature in question. The term typically indicates deviation from the indicated numerical value of ±10%, and preferably of ±5%.

Technical terms are used by their common sense. If a specific meaning is conveyed to certain terms, definitions of terms will be given in the following in the context of which the terms are used.

As described herein, "preferred embodiment" or "preferred aspect" means "preferred embodiment of the present invention" or "preferred aspect of the present invention". Likewise, as described herein, "an embodiments", "another embodiment", "an aspect", "another aspect" means "an embodiments of the present invention", "another embodiment of the present invention", "an aspect of the present invention" and "another aspect of the present invention", respectively.

For the purposes of the present invention, the term "obtained" is considered to be a preferred embodiment of the term "obtainable". If hereinafter e.g. an antibody is defined to be obtainable from a specific source, this is also to be understood to disclose an antibody which is obtained from this source.

Unless otherwise defined herein, scientific and technical terms used in connection with the present invention shall have the meanings that are commonly understood by those of ordinary skill in the art. The methods and techniques of the present invention are generally performed according to conventional methods well known in the art. Those skilled in the art will recognize, or be able to ascertain, using not more than routine experimentation, many equivalents to the specific embodiments of the invention described herein. Such equivalents are intended to be encompassed by the present invention.

### Examples

### Example 1

60,5 g deionized water and different amounts of biocatalyst (Table 1) was added to a thermostated reactor equipped with an agitator (EasyMax reactor system, Mettler Toledo). The biocatalyst consisted of spray dried whole cells of Rhodococcus rhodochrous, strain NCIMB 41164, with a specific nitrile hydratase activity of 103 kU/g, based on the complete dry biocatalyst powder (NHase activity assay is described below). The temperature was set to 20 °C, and this temperature was kept constant throughout the complete biocatalytic reaction. 39,5 g acrylonitrile was added to the reactor at a constant rate of 9,9 g/h. After 1 and 6 hours, a sample was taken and acrylamide, acrylonitrile and acrylic acid were analyzed by HPLC as described below. In all three reactions, complete bioconversion of acrylonitrile had been achieved within 6 hours, resulting in > 51 % acrylamide and < 100 ppm acrylonitrile content in the solution. The results referring to acrylic acid content are shown in table 1. "NHase activity in reaction" in Tables 1 and 2 is defined as the total amount of NHase activity added to the reactor divided by the total amount of acrylonitrile converted to acrylamide in the reaction.

**Table 1**

| **Biomass amount [mg]** | **Specific NHase activity [kU/g]** | **Nhase addition [U/g ACN]** | **Acrylic acid - 1h [ppm]** | **Acrylic acid - 6h [ppm]** |
|---|---|---|---|---|
| 61,7 | 103 | 161 | 382 | 489 |
| 66,9 | 103 | 174 | 382 | 514 |
| 72,0 | 103 | 188 | 393 | 625 |

### Example 2

The bioconverison reaction was carried out as described in example 1, except that the biocatalyst consisted of lyophilized whole cells of Rhodococcus rhodochrous, strain J1, with a specific nitrile hydratase activity of 157 kU/g, based on the complete dry biocatalyst powder (NHase activity assay is described below). The amounts of biocatalyst used are shown in table 2. In all three reactions, complete bioconversion of acrylonitrile had been achieved within 6 hours, resulting in > 51 % acrylamide and < 100 ppm acrylonitrile content in the solution. The results referring to acrylic acid content are shown in table 2.

**Table 2**

| **Biomass amount [mg]** | **Specific NHase activity [kU/g]** | **NHase addition [U/g ACN]** | **Acrylic acid - 1h [ppm]** | **Acrylic acid - 6h [ppm]** |
|---|---|---|---|---|
| 40,5 | 157 | 161 | 576 | 649 |
| 43,9 | 157 | 174 | 574 | 706 |
| 47,3 | 157 | 188 | 660 | 799 |

### Example 3 - NHase assay

The NHase activity of the above experiments has been determined by the following method: 100 µl of a cell suspension, cell lysate, dissolved enzyme powder or any other preparation containing the biocatalyst was contacted with 875 µl of an 50 mM potassium phosphate buffer and 25 µl of acrylonitrile at 25 °C on an eppendorf tube shaker at 1,000 rpm for 10 minutes. After 10 minutes of reaction time, samples were drawn and immediately quenched by adding the same volume of 1.4% hydrochloric acid. After mixing of the sample, cells were removed by centrifugation for 1 minute at 10,000 rpm and the amount of acrylamide formed was determined by analyzing the clear supernatant by HPLC. The concentration of acrylamide was between 0.25 and 1.25 mmol/l. The activity is deduced from the concentration of acrylamide by dividing the acrylamide concentration derived from HPLC analysis by the reaction time, which has been 10 minutes and by multiplying this value with the dilution factor between HPLC sample and original sample.

### Example 4 - HPLC

The following conditions were applied in order to determine the contents of acrylamide" acrylonitrile and acrylic acid using HPLC:

| | |
|---|---|
| Column: | Aqua C18 5 µm, 250*4.6 mm (Phenomenex) |
| Guard column: | C18 Aqua |
| Temperature: | 40 °C |
| Flow rate: | 1.00 ml/min |
| Injection volume: | 1.0 µl |
| Detection: | UV detector, wavelength 210 nm |
| Stop time: | 10.0 minutes |
| Post time: | 0.0 minutes |
| Maximum pressure: | 250 bar |
| Eluent A: | 10 mM KH2PO4, pH 2.5 |
| Eluent B: | Acetonitrile |
| Isocratic mode: | 90% Eluent A, 10% Eluent B |
| | |
| Matrix: | Fermentation broths, bioconversion mixtures |
| | Sample is filtered through 0.22 µm |

### Analytes:

| | Retention time [min] | Linear range [mg/l] |
|---|---|---|
| Acrylamide | 3.6 | 60-600 |
| Acrylic acid | 5,9 | 20-160 |
| Acrylonitrile | 6,8 | 1-40 |

## Claims

1. Method for producing acrylamide from acrylonitrile in an aqueous solution using a biocatalyst, wherein the ratio of the total amount of NHase fed to the reaction mixture by the addition of the biocatalyst to the total amount of acrylonitrile fed to the reaction mixture at the end of the reaction is less than 200 U/g acrylonitrile.

2. Method according to claim 1, wherein the ratio of the total amount of NHase fed to the reaction mixture by the addition of the biocatalyst to the total amount of acrylonitrile fed to the reaction mixture at the end of the reaction is equal or less than 188 U/g, preferably equal or less than 174 U/g, most preferably equal or less than 161 U/g acrylonitrile.

3. Method of claim 1 or 2, wherein the acrylic acid concentration of the reaction mixture at the end of the reaction is 1000 ppm or less, preferably less than 900 ppm or less, more preferably 750 ppm or less, even more preferably 650 ppm or less, most preferably 500 ppm or less based on the total amount of acrylamide produced in the reaction mixture.

4. Method according to any one of the preceding claims, wherein the reaction is carried out in fed-batch mode and wherein acrylonitrile is added to the reaction solution intermittently or continuously.

5. Method according to any one of the preceding claims, wherein the biocatalyst is treated by a drying step before being added to the reaction mixture.

6. Method according to claim 5, wherein the drying step is a spray drying step or freeze drying step, preferably spray drying step.

7. Method according to any one of the preceding claims, wherein acrylonitrile is added to the reaction mixture independently of the acrylonitrile concentration in the reaction mixture.

8. Method according to any one of the preceding claims, wherein the final acrylamide content in the reaction mixture is 40 w/w % to 60 w/w , preferably 45 w/w % to 60 w/w %, most preferably at least 50 w/w % to 60 w/w % based on the total weight of the reaction mixture.

9. Method according to any one of the preceding claims, wherein the biocatalyst is selected from the group consisting of *Rhodococcus, Aspergillus, Acidovorax, Agrobacterium, Bacillus, Bradyrhizobium, Burkholderia, Escherichia, Geobacillus, Klebsiella, Mesorhizobium, Moraxella, Pantoea, Pseudomonas, Rhizobium, Rhodopseudomonas, Serratia, Amycolatopsis, Arthrobacter, Brevibacterium, Corynebacterium, Microbacterium, Micrococcus, Nocardia, Pseudonocardia, Trichoderma, Myrothecium, Aureobasidium, Candida, Cryptococcus, Debaryomyces, Geotrichum, Hanseniaspora, Kluyveromyces, Pichia, Rhodotorula, Comomonas, and Pyrococcus,* preferably wherein the biocatalyst is selected from the group consisting of *Rhodococcus, Pseudomonas, Escherichia* and *Geobacillus.*

10. The method of claim 10, wherein the biocatalyst is *Rhodococcus rhodochrous* or *Rhodococcus pyridinovorans.*

11. Method for producing an acrylamide homopolymer or copolymer comprising the method of any one of claims 1 to 10 and further comprising the step of polymerizing the acrylamide obtained by claims 1 to 10.

12. Acrylamide homopolymer or copolymer obtainable by the method of claim 11.

13. Acrylamide homopolymer or copolymer, in particular according to claim 12, having an acrylic acid content of 60,000 ppm or less, preferably of 20,000 ppm or less, more preferably of 10,000 ppm or less, even more preferably of 2,000 ppm or less, and most preferably of 1,500 ppm or less, wherein the indications of ppm each relate to weight parts and are each referred to the total weight of the solid acrylamide homopolymer or copolymer.

14. Method for producing an aqueous acrylamide solution having a reduced acrylic acid concentration when compared to a reference solution by converting acrylonitrile to acrylamide using a biocatalyst, the ratio of the total amount of NHase fed to the reaction mixture by the addition of the biocatalyst to the total amount of acrylonitrile fed to the reaction mixture at the end of the reaction is less than 200 U/g acrylonitrile,
wherein the reference solution is an acrylamide solution which is prepared by the same method but the ratio of the total amount of NHase fed to the reaction mixture by the addition of the biocatalyst to the total amount of acrylonitrile fed to the reaction mixture at the end of the reaction is less than 200 U/g acrylonitrile.

15. Method according to claim 14, wherein the ratio of the total amount of NHase fed to the reaction mixture by the addition of the biocatalyst to the total amount of acrylonitrile fed to the reaction mixture at the end of the reaction is equal or less than 188 U/g, preferably equal or less than 174 U/g, most preferably equal or less than 161 U/g acrylonitrile.

16. Method according to claim 14 or 15, wherein the acrylic acid concentration when compared to a reference solution is reduced by at least 5 %, preferably at least 10 %, more preferably at least 15 %, even more preferably at least 18 %, most preferably at least 20 %.
